# EUROPÄISCHE PATENTSCHRIFT

(11) **EP 1 097 702 B1**
(45) Veröffentlichungstag und Bekanntmachung des Hinweises auf die Patenterteilung: **12.03.2008**
(21) Anmeldenummer: 00123932.6
(22) Anmeldetag: 03.11.2000
(51) Int. Cl.: A61K 8/73, A61K 8/81, A61K 8/86, A61Q 1/06, A61Q 1/10

(54) **Strukturierte Kosmetikmasse**
Structured cosmetic composition
Composition cosmétique structurée

(30) Priorität: 05.11.1999 DE 19953336
(43) Veröffentlichungstag der Anmeldung: 09.05.2001
(73) Patentinhaber: Schwan-STABILO Cosmetics GmbH & Co. KG, 90562 Heroldsberg (DE)
(72) Erfinder:
(74) Vertreter: Leissler-Gerstl, Gabriele

(56) Entgegenhaltungen:
- EP-A- 0 345 075
- EP-A- 0 615 744
- WO-A-95/31967
- WO-A-97/17055
- DE-A- 19 646 233
- JP-A- 8 245 363
- US-A- 4 332 763
- US-A- 5 208 028
- US-A- 5 756 437
- H. F. MASO: "Ingredients for Cosmetics and Toiletries - Chapter D" 20. Oktober 1999 (1999-10-20) , THE CENTER FOR PROFESSIONAL ADVANCEMENT , NEW JERSEY XP002161478 * Seite 21 * * Seite 26-31 *

## Beschreibung

Die vorliegende Erfindung betrifft eine strukturierte Kosmetikmasse sowie ein Verfahren zu ihrer Herstellung und ihre Verwendung.

Auf dem Gebiet der Kosmetik, insbesondere der dekorativen Kosmetik, werden häufig strukturierte Massen eingesetzt, die auf der Haut oder Schleimhaut verteilt oder verrieben werden und dort längere Zeit haften sollen. Derartige Massen finden beispielsweise in Form von Creme, Gel, gepresstem Puder oder in Stiftform Anwendung. Für die dekorative Kosmetik, insbesondere für Augen und Lippen, ist die Verwendung von Stiften beliebt, z.B. in Form von Lippenstiften, Augenbrauenstiften, Kajalstiften oder Lidschattenstiften. Für diesen Anwendungszweck muß die Masse fest genug sein, um Minen formen zu können, soll sich aber andererseits gut auftragen lassen und lange haften, ohne klebrig zu sein. Darüberhinaus soll sie möglichst noch wasserfest sein und die Haut nicht reizen. Wenn als Stift ein Drehstift mit einer Drehmechanik, in die die Mine eingesetzt wird und freitragend ist, zum Einsatz kommt, muß die Masse darüberhinaus auch eine ausreichende Festigkeit haben.

Zur Herstellung von Stiften gibt es bereits zahlreiche Rezepturen. So werden z.B. Stiftmassen beschrieben und hergestellt, die flüchtige Lösungsmittel enthalten, die bei Körpertemperatur verdampfen. Das flüchtige Lösungsmittel bewirkt, dass sich die Masse leicht auftragen läßt. Nach dem Verdampfen des Lösungsmittels nach dem Auftragen auf die Haut haftet die Masse gut auf der Haut. Allerdings ist die Verwendung flüchtiger Lösungsmittel in letzter Zeit aus Umweltgründen unerwünscht. Wünschenswert wäre daher die Bereitstellung einer Masse, die mit Wasser als Lösungsmittel auskommt.

Es wurden bereits wasserhaltige Stiftmassen beschrieben, die in Form wasserhaltiger Gele vorliegen. So beschreibt beispielsweise WO97/17055 Gelstifte, die einen hohen Anteil an Wasser enthalten können, allerdings auch mindestens 20 Gew.-% eines wasserlöslichen oder wasserdispergierbaren Gelbildners enthalten müssen. Der hohe Anteil an Gelbildner erzeugt beim Auftragen ein klebriges, unangenehmens Gefühl.

Auch DE 196 432 37 beschreibt Stifte, die einen Wassergehalt von 30 bis 85 Gew.-% enthalten können. Hier wird die Stiftmasse in Form einer festen W/O-Emulsion hergestellt. Bei diesen Massen wird die Wasserphase dazu genutzt, in Wasser, aber nicht in der Fettphase lösliche Substanzen in einen Stift einzuarbeiten. Die hier beschriebenen Produkte befriedigen jedoch noch nicht, da sie bei höheren Temperaturen nicht stabil sind und sich in die Phasen auftrennen.

Aufgabe der vorliegenden Erfindung war es daher, eine Kosmetikmasse bereitzustellen, die in strukturierter Form vorliegt, zu Stiften verarbeitet werden kann, die gute mechanische Eigenschaften aufweist, die eine hohe Wassermenge aufnehmen kann, äußerst temperaturstabil ist, in einem breiten pH-Bereich stabil ist und ohne Veränderung bis zu etwa 60°C lagerstabil und bis 45°C auftragbar ist. Weiterhin soll die Kosmetikmasse wischfest sein, dabei aber leicht gleichmäßig aufgetragen werden können und ein gutes Hautgefühl ergeben. Außerdem soll sie farbkräftige Massen für die dekorative Kosmetik liefern.

Diese Aufgabe wird gelöst mit einer strukturierten Kosmetikmasse, die Wasser, eine Fettkomponente, einen O/W-Emulgator, einen W/O-Emulgator, κ-Carraghen, sowie gegebenenfalls übliche Inhaltsstoffe umfaßt.

Es wurde gefunden, dass erfindungsgemäß eine äußerst temperaturstabile strukturierte Masse erhalten werden kann, wenn eine Dispersion aus einer Wasserkomponente und einer Fettkomponente mit einer Kombination eines W/O-Emulgators und eines O/W-Emulgators vereinigt wird und dieser Zusammensetzung κ-Carraghen, zugesetzt wird. Bei der erfindungsgemäßen Masse handelt es sich um einen Dispersionstyp, der eine kontinuierliche wäßrige Phase aufweist, im wesentlichen aber keine Emulsionsmerkmale erkennen läßt. Der Zusatz des speziellen Polysaccharids erzeugt eine feste Struktur, so dass die Masse geformt werden kann, beispielsweise zu Minen verarbeitet werden kann, die sowohl in Stifthülsen als auch die Drehmechanik von Drehstiften eingesetzt werden können.

Die erfindungsgemäße Masse umfaßt als Hauptkomponenten Wasser und eine Fettkomponente. Wasser kann in der Masse in einem Anteil von 30 bis 85 Gew.-%, bevorzugt 40 bis 75 Gew.-% und besonders bevorzugt 50 bis 65 Gew.-%, jeweils bezogen auf das Gewicht der gesamten Zusammensetzung, enthalten sein. Ein Teil des Wassers verdampft beim Auftragen auf die Haut und erzeugt ein angenehm kühlendes Hautgefühl.

Die Fettkomponente der erfindungsgemäßen Kosmetikmasse besteht aus Öl- und/oder Wachskomponenten. Hier sind alle auf dem Gebiet der Kosmetik üblicherweise verwendeten Öle und Wachse geeignet. Die Auswahl richtet sich danach, wofür die Kosmetikmasse eingesetzt werden soll. Geeignet als Ölkomponente sind natürliche und synthetische Öle, z.B. pflanzliche Öle und Esteröle oder Silikonöle. Als Beispiele für Ölkomponenten können Jojobaöl, Rizinusöl, Olivenöl und pflanzliche Triglyceridöle genannt werden. Beispiele für Esteröle sind Jojobate, Myristylmyristat, Isopropylmyristat und Isopropylpalmitat. Als Silikonöle können sowohl flüchtige als auch nicht flüchtige Siliconöle eingesetzt werden. Für Stifte mit härterer Textur, wie z.B. Augenbrauenstifte und Kajalstifte werden bevorzugt Paraffin, Ceresin, Ozokerite und mikrokristalline Wachse verwendet. Als Wachskomponente kommen die in der Kosmetik üblichen Wachse inbetracht. Ein Beispiel ist Polyethylenwachs.

Erfindungswesentlich ist die Kombination aus O/W-Emulgator und W/O-Emulgator. Die ausgewählten Emulgatoren an sich sind nicht kritisch und es können die üblicherweise in Kosmetikmassen verwendeten Emulgatoren eingesetzt werden. Es ist jedoch wesentlich, dass von jeder der beiden Klassen mindestens ein Vertreter in der Masse vorhanden ist. Das Verhältnis zwischen den beiden Emulgatoren ist bevorzugt 1:5 bis 5:1, besonders bevorzugt 1:2 bis 2:1. Als O/W-Emulgatoren werden bevorzugt langkettige Ester von mehrwertigen Alkoholen, z.B. langkettige Ester von Glycerin und Saccharose verwendet, wobei die Alkoholanteile bevorzugt 14 bis 26 Kohlenstoffatome, insbesondere 18 bis 24 Kohlenstoffatome aufweisen.

Als W/O-Emulgatoren werden bevorzugt Ethylenoxidderivate, wie PEG-30-Dipolyhydroxystearat oder Emulgatoren aus der Gruppe der Cetearethe, sowie höhere Alkohole, z.B. solche mit 20 bis 40 C-Atomen verwendet. Beispiele sind Polyoxyethylen-20; Polyoxyethylen-30; Ceteareth-20; Ceteareth-30; Polyoxyethylen-24-Glycerinmonostearat sowie Polyoxyethylen-10-Oleylcetyalkohol oder Mischungen davon.

Ein weiterer erfindungswesentlicher Bestandteil der erfindungsgemäßen Kosmetikmasse ist ein Polysaccharid, das ein dreidimensionales Netz bilden kann. Welche Polysaccharide hier geeignet sind, kann der Fachmann mit wenigen Versuchen feststellen, indem er überprüft, welche Art von Gelstruktur ein inbetracht gezogenes Polysaccharid ausbildet.

Die Carraghene, auch als Carrageenane bezeichnet, werden aus Rotalgen, insbesondere solchen der Familien Gigartinaceae oder Solieriaceae gewonnen und sind komplexe Gemische verschiedener Polysaccharide. Durch fraktionierte Fällung können verschiedene Komponenten des Carraghens gewonnen werden, die in Klassen eingeteilt werden können; eine davon ist *κ*-Carraghen.

Überraschenderweise wurde gefunden, dass *κ*-Carraghen der erfindungsgemäßen Dispersion eine feste Struktur verleihen können, während Polysaccharide, die zweidimensionale Netze bilden, z.B. andere Carraghenarten wie Jota-Carraghen oder Lambda-Carraghen, nur streichfähige pastöse Produkte liefern, die sich nicht zu strukturierten Massen, insbesondere nicht zu Stiften verarbeiten lassen. Ein Polysaccharid, wie oben definiert, führt in Kombination mit dem O/W-Emulgator und dem W/O-Emulgator in der Dispersion zu einer Struktur, die auch bei relativ hohen Temperaturen stabil bleibt.

Erfindungsgemäß wird als Polysaccharid *κ*-Carraghen verwendet. Das Polysaccharid wird bevorzugt in einem Anteil von 0,05 bis 18 Gew.-% eingesetzt. Eine geringere Menge führt nicht mehr zu den vorteilhaften Eigenschaften, während eine über 18 Gew.-% hinausgehende Menge keine weiteren Vorteile erbringt, aber ein unangenehm klebendes Gefühl erzeugen kann. Besonders bevorzugt wird das Polysaccharid in einer Menge von 0,1 bis 10 Gew.-% und insbesondere in einer Menge von 0,3 bis 4 Gew.-% eingesetzt.

Die erfindungswesentlichen Merkmale sind somit der Einsatz einer Kombination aus O/W-Emulgator und W/O-Emulgator und der Zusatz des Polysaccharids, insbesondere von *κ*-Carraghen. Nur bei Verwendung dieser Kombination für eine Wasser und Fettkomponenten enthaltende Dispersion erhält man eine strukturierte Masse mit den ausgeführten wünschenswerten Eigenschaften.

Die erfindungsgemäße Kosmetikmasse kann darüber hinaus weitere übliche Inhaltsstoffe enthalten. Üblicherweise enthält sie Farbstoffe, Farblacke, Pigmente und/oder Perlglanzmittel, um der Masse das gewünschte Aussehen zu verleihen. Es können erfindungsgemäß alle Färbemittel in der in der Kosmetik üblichen und durch die jeweils gesetzlichen Regelungen der einzelnen Länder zugelassenen Art und Menge eingesetzt werden. Da die Kosmetikmasse einen Anteil an Wasser enthält, können wasserlösliche und wasserdispergierbare Farbstoffe vorteilhaft eingesetzt werden.

Weiterhin ist es üblich, Kosmetikmassen Verdickungsmittel bzw. Füllstoffe zuzusetzen, um die Konsistenz einzustellen und die Struktur zu beeinflussen. Alle hier üblichen Füllstoffe und Verdickungsmittel sind geeignet. Beispielsweise können Kaolin, Talkum, modifizierte Stärken verwendet werden. Die erfindungsgemäße Kosmetikmasse kann auch Mittel zur Verbesserung der Struktur und zur Einstellung einer besseren Abgabe auf der Haut enthalten. Als Beispiele hierfür können Bornitrit, Lauroyllysin, modifizierte Glimmerarten, Nylon 12 und PMMA genannt werden. Diese Zusatzstoffe werden in der für Kosmetikmassen üblichen Menge eingesetzt. Auch Antioxidantien und antimikrobielle Mittel können zur Erhöhung der Stabilität in den an sich üblichen Mengen in der erfindungsgemäßen Kosmetikmasse enthalten sein. Weiterhin können übliche Stabilisatoren, z.B. Kester-Wachs oder C₂₀-C₄₀-Alkohole in den Kosmetikmassen enthalten sein. Auch ein Feuchthaltemittel, z.B. ein mehrwertiger Alkohol, wie Sorbit, Glycerin oder Propylenglykol, kann in üblicher Menge zugegeben werden.

Ein weiterer Gegenstand der Erfindung ist ein Verfahren zur Herstellung einer festen Kosmetikmasse, dass dadurch gekennzeichnet, dass man die Fettbestandteile und die Emulgatoren erwärmt, bis sie dünnflüssig sind, gegebenenfalls Pigmente und Füllstoffe zusetzt, getrennt davon das Polysaccharid in Wasser löst, bei einer Temperatur im Bereich von 50 bis 100°C die Fett- und die Wasserphase vereinigt, homogenisiert, entlüftet, abkühlt, gegebenenfalls thermisch labile Bestandteile zusetzt und anschließend lagert

Da die erfindungsgemäße Kosmetikmasse sowohl eine Wasser- als auch eine Fettkomponente aufweist, können alle in der Kosmetik üblichen wasserlöslichen oder wasserdispergierbaren sowie fettlöslichen Zusätze ohne Probleme eingesetzt werden. Allerdings ist die Verwendung von hitzelabilen Rohstoffen, d.h. Materialien, die Temperaturen von mehr als 60°C nicht aushalten, weniger wünschenswert. Da die Herstellung der erfindungsgemäßen Kosmetikmassen geeigneterweise bei einer Temperatur im Bereich von 50 bis 100°C erfolgt, ist es bevorzugt, solche Rohstoffe einzusetzen, die Temperaturen in diesem Bereich aushalten. Wenn hitzelabile Materialien verwendet werden, so müssen diese am Ende des Herstellungsverfahrens eingearbeitet werden.

Die erfindungsgemäße feste Kosmetikmasse wird hergestellt, indem die Fettbestandteile erwärmt werden, bis sie dünnflüssig sind und dann die fettlöslichen oder fettdispergierbaren Bestandteile zugesetzt werden und gegebenenfalls homogenisiert werden. Dann wird das Polysaccharid in der Wasserphase in der Wärme gelöst. Gegebenenfalls können wasserlösliche und wasserdispergierbare Inhaltsstoffe zugesetzt werden. Die Fettphase und die Wasserphase werden dann bei einer Temperatur im Bereich von 50 bis 100°C, bevorzugt 60 bis 80°C, insbesondere 70 bis 75°C vereinigt, homogenisiert und entlüftet. Anschließend können temperaturlabile Substanzen, z.B. Antioxidantien, Konservierungsmittel und Pflegestoffe zugesetzt werden. Falls derartige Materialien zugesetzt werden, wird die Masse nochmals kurz homogenisiert. Anschließend wird die Masse abgekühlt und kann in dieser Form gelagert werden. Sie ist über mehrere Monate stabil, ohne dass sich die Phasen trennen.

Zur Herstellung von Stiften kann die Masse, ohne Abkühlung, in vorbereitete Hülsen eingegossen werden oder zu Minen geformt werden, die dann in eine Hülse oder die Drehmechanik eines Drehstiftes eingesetzt werden. Bevorzugt werden Minen mit einem Durchmesser von 2 bis 12 mm geformt.

Besonders bevorzugt wird die erfindungsgemäße Kosmetikmasse zur Herstellung von Lippenstiften, Liplinern, Lidschattenstiften, Eyelinern, Augenbrauenstiften, Sonnenschutzstiften und Deo- oder Antiperspirant-Stiften verwendet. Je nach dem Anwendungszweck können der Kosmetikmasse die jeweils notwendigen weiteren Bestandteile zugesetzt werden, d.h. im Fall von dekorativer Kosmetik Farbstoffe und Glanzpigmente, im Fall von Sonnenschutzstiften Sun-Blocker mit Lichtschutzfaktor und im Fall von Deostift oder Antiperspirant-Stiften, Parfums und schweißhemmende Mittel. Der Fachmann kennt die jeweils geeigneten Einsatzstoffe, so dass diese hier keiner weiteren Erläuterung bedürfen.

Im folgenden werden noch einige Beispiele angegeben, um die Erfindung weiter zu erläutern. Die Rohstoffe werden dabei mit den üblichen INCI-Namen bezeichnet.

### Beispiel 1

Im folgenden ist die Rezeptur für einen erfindungsgemäßen Eyeliner angegeben. Die Bestandteile sind mit den üblichen INCI-Namen bezeichnet und die Mengen sind in Gewichtsprozent jeweils bezogen auf die Gesamtzusammensetzung angegeben.

| | |
|---|---|
| Aqua | 60,000 |
| Colorants | 12,000 |
| Sorbitol Solution | 7,000 |
| Cetearyl Behenate | 6,500 |
| Cyclomethicone | 6,000 |
| PVP Eicosene Copolymer | 3,500 |
| Ceteareth-16 | 2,000 |
| PEG-30 Dipolyhydroxystearate | 2,000 |
| Chondrus Crispus (κ-Carraghen) | 1,000 |
| | 100,000 |

Die Fettbestandteile, wurden zusammen mit den Emulgatoren auf 70 - 75°C erwärmt und die Colorants und Füllstoffe zugefügt. Das Carraghen wurde in Wasser in der Wärme gelöst. Beide Phasen wurden dann vereinigt, homogenisiert und entlüftet. Die Masse wurde zu einer Mine geformt und in eine Hülse eingesetzt. Der erhaltene Eyelinerstift ließ sich leicht auftragen und ein aufgetragener Strich haftete gut auf der Haut.

### Beispiel 2

Im folgenden ist die Rezeptur für einen erfindungsgemäßen Augenbrauenstift angegeben. Die Bestandteile sind mit den üblichen INCI-Namen bezeichnet und die Mengen sind in Gewichtsprozent jeweils bezogen auf die Gesamtzusammensetzung angegeben.

| | |
|---|---|
| Aqua | 65,000 |
| Colorants | 10,000 |
| Cyclomethicone | 6,000 |
| Ceteareth-16 | 2,000 |
| PEG-30 Dipolyhydroxystearate | 2,000 |
| Chondrus Crispus (κ-Carraghen) | 1,000 |
| Glycerin | 4,000 |
| Polyethylene | 7,000 |
| PVP/Hexadecene Copolymer | 3,000 |
| | 100,000 |

Die Herstellung des Stiftes erfolgte wie in Beispiel 1 beschrieben. Der erhaltene Stift ließ sich gut auftragen und war auch bei längerer Lagerung stabil.

### Beispiel 3

Im folgenden ist die Rezeptur für einen erfindungsgemäßen Kajalstift angegeben. Die Bestandteile sind mit den üblichen INCI-Namen bezeichnet und die Mengen sind in Gewichtsprozent jeweils bezogen auf die Gesamtzusammensetzung angegeben.

| | |
|---|---|
| Aqua | 65,000 |
| Colorants | 12,000 |
| PVP Eicosene Copolymer | 2,500 |
| Chondrus Crispus (κ-Carraghen) | 1,000 |
| Isostearylalcohol | 6,000 |
| Propylene Glycol | 6,000 |
| Sucrose Tetrastearate Triacetate | 3,500 |
| PVP/Hexadecene Copolymer | 1,500 |
| C20-C40 Alcohols | 5,000 |
| | 100,000 |

Die Herstellung des Stiftes erfolgte wie in Beispiel 1 beschrieben. Der erhaltene Stift ließ sich gut auftragen und wurde als angenehm empfunden.

### Beispiel 4

Im folgenden ist die Rezeptur für einen erfindungsgemäßen Lipliner angegeben. Die Bestandteile sind mit den üblichen INCI-Namen bezeichnet und die Mengen sind in Gewichtsprozent jeweils bezogen auf die Gesamtzusammensetzung angegeben.

| | |
|---|---|
| Aqua | 65,000 |
| Colorants | 12,000 |
| Sorbitol Solution | 6,000 |
| Chondrus Crispus (κ-Carraghen) | 1,000 |
| Sucrose Tribehenate | 4,000 |
| Sucrose Polyinoleate | 2,000 |
| Dimethicone | 2,000 |
| C20-C40 Alcohols | 6,000 |
| Jojoba Oil | 2,000 |
| | 100,000 |

Die Herstellung des Stiftes erfolgte wie in Beispiel 1 beschrieben. Mit dem erhaltenen Stift ließen sich Linien gut ziehen und hafteten gut auf der Haut.

### Beispiel 5

Im folgenden ist die Rezeptur für einen erfindungsgemäßen Lipliner angegeben. Die Bestandteile sind mit den üblichen INCI-Namen bezeichnet und die Mengen sind in Gewichtsprozent jeweils bezogen auf die Gesamtzusammensetzung angegeben.

| | |
|---|---|
| Aqua | 65,000 |
| Colorants | 12,000 |
| Cyclomethicone | 6,000 |
| PEG-30 Dipolyhydroxystearate | 2,000 |
| Chondrus Crispus (κ-Carraghen) | 1,000 |
| Glycerin | 5,000 |
| Sucrose Tribehenate | 4,000 |
| C20-C40 Alcohols | 2,000 |
| Jojoba Oil | 3,000 |
| | 100.000 |

Die Herstellung des Stifts erfolgte wie in Beispiel 1 beschrieben. Die mit der Masse hergestellte Mine war fest genug, daß sie in eine Drehmechanik eingesetzt werden konnte und herausgedreht werden konnte, ohne abzubrechen.

## Patentansprüche

1. Strukturierte Kosmetik-Minenmasse umfassend Wasser, eine Fettphase, einen O/W-Emulgator, einen W/O-Emulgator, κ-Carraghen, sowie ggf. übliche Inhaltsstoffe.

2. Kosmetik-Minenmasse nach Anspruch 1,
**dadurch gekennzeichnet, dass** sie als Fettphase Öl- und/oder Wachskomponenten enthält.

3. Kosmetik-Minenmasse nach einem der vorhergehenden Ansprüche,
**dadurch gekennzeichnet, dass** sie 30 bis 85 Gew.-% Wasser, 0,05 bis 18 Gew.-% Polysaccharid, 2 bis 15 Gew.-% O/W-Emulgator und W/O-Emulgator und Rest Fettphase enthält.

4. Kosmetik-Minenmasse nach einem der vorhergehenden Ansprüche,
**dadurch gekennzeichnet, dass** die Fettphase 10-25 Gew.-% der Kosmetik-Minenmasse bildet.

5. Kosmetik-Minenmasse nach einem der vorhergehenden Ansprüche,
**dadurch gekennzeichnet, dass** ONV-Emulgator und W/O-Emulgator in einem Gewichtsverhältnis von 1:5 bis 5:1 vorliegen.

6. Kosmetik-Minenmasse nach einem der vorhergehenden Ansprüche,
**dadurch gekennzeichnet, dass** sie κ-Carraghen in einem Anteil von 0,1 bis 10 Gew.-%, bevorzugt 0,3 bis 4 Gew.-% enthält.

7. Kosmetik-Minenmasse nach einem der vorhergehenden Ansprüche,
**dadurch gekennzeichnet, dass** sie zusätzlich für kosmetische Massen übliche Inhaltsstoffe, insbesondere Antioxidantien, Konservierungsmittel, Füllstoffe, Farbstoffe und Perlglanzpigmente enthält.

8. Verfahren zur Herstellung einer festen Kosmetik-Minenmasse nach Anspruch 1,
**dadurch gekennzeichnet, dass** man die Fettbestandteile und die Emulgatoren erwärmt, bis sie dünnflüssig sind, gegebenenfalls Pigmente und Füllstoffe zusetzt, getrennt davon das Polysaccharid in Wasser löst, bei einer Temperatur im Bereich von 50 bis 100°C die Fett- und die Wasserphase vereinigt, homogenisiert, entlüftet, abkühlt, gegebenenfalls thermisch labile Bestandteile zusetzt und anschließend lagert.

9. Verfahren nach Anspruch 8,
**dadurch gekennzeichnet, dass** man die Kosmetik-Minenmasse in an sich bekannter Weise zu Minen verarbeitet.

10. Verfahren nach Anspruch 8 oder Anspruch 9,
**dadurch gekennzeichnet, dass** man die Kosmetik-Minenmasse entweder in eine Hülse gießt oder zu einer Mine formt und diese in eine Hülse oder die Drehmechanik eines Drehstiftes einsetzt.

11. Verwendung einer Kosmetik-Minenmasse nach einem der Ansprüche 1 bis 7 oder einer Masse, die mit einem Verfahren der Ansprüche 8 bis 10 hergestellt wurde, zur Herstellung von Lippenstiften, Liplinern, Lidschattenstiften, Eyelineren, Augenbrauenstiften, Sonnenschutzstiften und/oder Deo- oder Antiperspirantstiften.

## Claims

1. A structured cosmetic core composition comprising water, a fatty phase, an O/W emulsifier, a W/O emulsifier, κ-carrageen, together optionally with conventional ingredients.

2. A cosmetic core composition according to claim 1,
**characterised in that** it contains oil and/or wax components as the fatty phase.

3. A cosmetic core composition according to one of the preceding claims,
**characterised in that** it contains 30 to 85 wt.% water, 0.05 to 18 wt.% polysaccharide, 2 to 15 wt.% O/W emulsifier and W/O emulsifier, with the remainder being fatty phase.

4. A cosmetic core composition according to one of the preceding claims,
**characterised in that** the fatty phase forms 10-25 wt.% of the cosmetic core composition.

5. A cosmetic core composition according to one of the preceding claims,
**characterised in that** O/W emulsifier and W/O emulsifier are present in a weight ratio of from 1:5 to 5:1.

6. A cosmetic core composition according to one of the preceding claims,
**characterised in that** it contains κ-carrageen in a proportion of 0.1 to 10 wt.%, preferably 0.3 to 4 wt.%.

7. A cosmetic core composition according to one of the preceding claims,
**characterised in that** it additionally contains ingredients conventional for cosmetic compositions, in particular antioxidants, preservatives, fillers, dyes and pearlescent pigments.

8. A method of producing a solid cosmetic core composition according to claim 1,
**characterised in that** the fatty constituents and the emulsifiers are heated until they are highly fluid, pigments and fillers are optionally added, the polysaccharide is separately dissolved in water, at a temperature in the range from 50 to 100°C the fatty and aqueous phases are combined, homogenised, deaerated, cooled, heat-labile constituents are optionally added and the composition is then stored.

9. A method according to claim 8,
**characterised in that** the cosmetic core composition is processed in a manner known per se to form cores.

10. A method according to claim 8 or claim 9,
**characterised in that** the cosmetic core composition is either poured into a casing or shaped into a core, the latter being inserted into a casing or the rotary mechanism of a rotary stick.

11. Use of a cosmetic core composition according to one of claims 1 to 7 or of a composition produced using a method of claims 8 to 10 to produce lipsticks, lip liners, eye shadow pencils, eye liners, eyebrow pencils, sun protection sticks and/or deodorant or antiperspirant sticks.

## Revendications

1. Composition cosmétique structurée pour mines comprenant de l'eau, une phase grasse, un émulsifiant huile-dans-eau, un émulsifiant eau-dans-huile, du κ-carragaheen, ainsi que des ingrédients ou constituants usuels.

2. Composition cosmétique pour mines selon la revendication 1, **caractérisée en ce qu'**elle contient en tant que phase grasse des composantes d'huile et/ou de cire.

3. Composition cosmétique pour mines selon l'une des revendications précédentes, **caractérisée en ce qu'**elle contient 30 à 85 % en masse d'eau, 0,05 à 18 % en masse de polysaccharide, 2 à 15 % en masse d'émulsifiant huile-dans-eau et d'émulsifiant eau-dans-huile, et le reste de la phase grasse.

4. Composition cosmétique pour mines selon l'une des revendications précédentes, **caractérisée en ce que** la phase grasse forme 10 à 25 % en masse de la composition cosmétique pour mines.

5. Composition cosmétique pour mines selon l'une des revendications précédentes, **caractérisée en ce que** l'émulsifiant huile-dans-eau et l'émulsifiant eau-dans-huile sont présents selon un rapport de masse compris entre 1:5 et 5:1.

6. Composition cosmétique pour mines selon l'une des revendications précédentes, **caractérisée en ce qu'**elle contient du κ-carragahen selon une fraction comprise entre 0,1 et 10 % en masse (en poids), de préférence entre 0,3 et 4 % en masse (en poids).

7. Composition cosmétique pour mines selon l'une des revendications précédentes, **caractérisée en ce qu'**elle contient en outre des constituants usuels pour des compositions cosmétiques, en particulier des antioxydants, des agents conservateurs, des matières de charge, des colorants et des pigments perlés.

8. Procédé de fabrication d'une composition cosmétique solide pour mines selon la revendication 1, **caractérisé en ce que** l'on chauffe les constituants gras et les émulsifiants jusqu'à ce qu'ils soient fluides, que l'on ajoute éventuellement les pigments et les matières de charge, que l'on dissout séparément de cela le polysaccharide dans de l'eau, que l'on réunisse à une température dans la gamme de 50 à 100 °C la phase grasse et la phase aqueuse, que l'on homogénéise, que l'on désaère, que l'on refroidisse, que l'on ajoute éventuellement les constituants thermiquement labiles et que l'on stocke ensuite le tout.

9. Procédé selon la revendication 8, **caractérisé en ce que** l'on transforme en mines la composition cosmétique pour mines d'une façon connue en soi.

10. Procédé selon la revendication 8 ou la revendication 9, **caractérisé en ce que** l'on verse la composition cosmétique pour mines dans une gaine ou douille, ou qu'on la forme en une mine puis qu'on utilise celle-ci dans une gaine ou douille ou dans le mécanisme à rotation d'un bâtonnet ou crayon rotatif.

11. Utilisation d'une composition cosmétique pour mines selon l'une des revendications 1 à 7 ou d'une composition qui a été fabriquée selon un procédé des revendications 8 à 10, pour fabriquer des rouges à lèvres, des crayons à lèvres, des crayons de fard à paupière, des eyeliners, des crayons à sourcils, des sticks ou bâtonnets de protection solaire et/ou des sticks ou bâtonnets déodorants ou antiperspirants.
